# EUROPEAN PATENT APPLICATION

(11) **EP 2 141 241 A1**
(43) Date of publication of application: **06.01.2010**
(21) Application number: 08012010.8
(22) Date of filing: 03.07.2008
(51) Int. Cl.: C12N 15/861

(54) **Senescence cells and methods for its production**

(71) Applicant: Helmholtz-Zentrum für Infektionsforschung GmbH, 38124 Braunschweig (DE)
(72) Inventor: May, Tobias Dr., 38124 Braunschweig (DE); Wirth, Dagmar Dr., 38124 Braunschweig (DE); Hauser, Hansjörg Dr., 38302 Wolfenbüttel (DE)
(74) Representative: Kröncke, Rolf

(57) **Abstract**

The present invention relates to a method for the generation of transduced cell lines susceptible to senescence whereby the senescence is exogenously inducible. In particular, the present invention relates to a method for the generation of transduced cell lines susceptible to senescence wherein two or more different immortalizing gene sequences have been incorporated whereby said immortalizing gene sequences are regulated by regulators controlled via exogenous means. In a further aspect, the present invention relates to transduced cell lines obtainable with said method. In addition, methods for screening molecules influencing senescence of cells are provided as well as kits for conducting the same.

## Description

The present invention relates to a method for the generation of transduced cell lines susceptible to senescence whereby the senescence is exogenously inducible. In particular, the present invention relates to a method for the generation of transduced cell lines susceptible to senescence wherein two or more different immortalizing gene sequences have been incorporated whereby said immortalizing gene sequences are regulated by regulators controlled via exogenous means. In a further aspect, the present invention relates to transduced cell line obtainable with said method. In addition, methods for screening molecules influencing senescence of cells are provided as well as kits for conducting the same.

### Background art

Cellular senescence was described decades ago by Hayflick and Moorhead (HAYFLICK, and MOORHEAD. 1961, Exp.Cell Res. 25: 585-621). They demonstrated that normal human cells (fibroblasts) have a limited ability to proliferate in culture. After a certain number of population doublings - the number of cell divisions depends on the cell typo - the cell proliferation declines gradually. Eventually, all cells lose the ability to divide but remain viable and metabolically active for many weeks.

Two hypotheses arose that tried to describe the existence of such a phenomenon. The first one argued that senescence is a tumor suppressor mechanism. This stems mainly from the fact that cancer cells often proliferate indefinitely. Any means that suppress or counteract this proliferation is advantageous. In contrast to apoptosis which kills and completely eradicates unwanted cancer cells, senescence leads to a permanent growth arrest which hinders the cancer cells from overgrowing the normal cells though senescent cells are still viable.

The second hypothesis sees the effects of senescence less beneficial. With increasing age tissue regeneration and repair is greatly impaired. This fact was ascribed to senescent (stem) cells that lose with increasing age their regenerative potential.

Thus, it seems that senescence is a double-edged sword. On the one hand senescence fights against tumor development, on the other hand it impairs tissue regeneration. A deeper understanding of this phenomenon is mandatory to utilize it for the treatment of patients. It could be envisioned that certain senescence pathways can be activated that target cancer cells exclusively whereas other pathways could be suppressed to strengthen the tissue regeneration capacity.

The major barrier for targeted therapies is the complexity of the senescence phenomenon. This complexity results from the diversity of senescence stimuli that induce a plethora of different signalling cascades. Further complicating are (1) the differences in the senescence programs that are induced in different cell types and (2) the differcences between murine and human senescence mechanisms.

The stimuli that induce senescence can be divided into three different groups. The mechanism that was elucidated at first is telomere shortening. Telomeres are stretches of repetitive DNA at the very end of the linear chromosomes. These stretches cannot be replicated by DNA polymerases during replication. This leads to the "end-replication problem" which means that the cells lose roughly 200 base pairs of telomeric DNA during every replication phase. This telomere shortening proceeds until the telomeres reach a critical length. These short telomeres are sensed by the cells and induce a permanent growth arrest/senescence.

Another stimulus that induces senescence is DNA damage which can be for example induced by irradiation or chemotherapeutic drugs. On the molecular level this program strongly depends on p53 and its effector p21. However the downstream effectors of either p53 or p21 are still not unravelled. And even more importantly p53 or p21 can - depending on the context - also induce apoptosis or a transient growth arrest thereby further complicating the elucidation of the senescence effectors.

The third stimulus that is known to induce senescence is aberrant oncogenic signalling. For example overexpression of the oncogenic form of ras and downstream effectors like RAF, MEK, MOS or BRAF were found to induce a permanent growth arrest. Importantly, this senescence stimulus does not induce telomere shortening. And in contrast to DNA damage and telomere shortening the p16/pRB pathway is a major effector of oncogenic induced senescence. A problem is that this senescence program is in some cell types only induced when the oncogene is expressed at very high levels. This raised some scepticism about the relevance of the observed phenomenon.

An issue that complicates the elucidation of the senescence program is that there are marked differences between senescence pathways in different cell types (Zhang et al., 2003, Proc.Natl.Acad.Sci.U.S.A 100: 3251-3256). The differences include different senescence effectors as well as different stimuli utilized for the senescence induction. For example a study demonstrated that the gene expression profile between fibroblasts and mammary epithelial cells differs greatly and shows no significant overlap. In addition the stimuli required for senescence induction differ in both cell types. The mammary epithelial cells stop their proliferation after a few passages and undergo a permanent growth arrest. This phenomenon is independent from telomere shortening and highly dependent on the p16/pRB pathway. However, special culture conditions can prevent the cells from entering this permanent growth arrest. Using these culture conditions, the mammary epithelial cells proliferate until their telomeres reach a critical length which then induces a different senescence program. Fibroblasts on the other hand senesce heterogeneously on the molecular level (comparable to a mosaic). While in some cells telomere shortening is the trigger, the p16/pRB pathway triggers senescence in others.

The model organism that is mainly used for genetic studies is the mouse because of the high genetic similarity between mice and humans and the ease of manipulation of the murine genome. Unfortunately, the senescence program between the two species differs greatly in several aspects. For example in contrast to their human counterparts primary murine fibroblasts cease their proliferation and undergo a permanent growth arrest despite the fact that they possess long telomeres. Another marker for senescent cells is the formation of the so-called senescence associated heterochromatin foci (SAHF). The formation of these foci is triggered by the p16/pRB pathway. In contrast to human cells - where only senescent cells form these foci - murine cells form these foci independent of their proliferation status. On the molecular level these differences are highlighted by the fact that for senescence induction in murine cells mainly the p53 pathway is used whereas in human cells both the p53 and the p16/pRB pathways can be employed for the induction of senescence.

This complexity results in a severe problem. Up to now marker(s) are missing that clearly identify senescent cells. Among the commonly used markers are SA-β-Gal (senescence-associated-beta galactosidase) staining (Dimri et al., 1995, Proc.Natl.Acad.Sci.U.S.A 92: 9363-9367), the formation of SAHFs, a permanent growth arrest, a change in morphology to a more flattened and spread morphology and an increase in granularity which is probably due to the increase in lysosomes (reviewed in Campisi,J. and d.F d'Adda. 2007. Nat.Rev.MoLCell Biol. 8: 729-740). The universally accepted marker SA-β-Gal is a enzymatic activity that converts X-Gal (5-Brom-4-chlor-3-indoxyl-β-D-galactopyranosid) at a pH 6 and leads to blue staining of senescent cells. This enzymatic activity is linked to the lysosomes and probably reflects the increase in lysosome number. However, the SA-β-Gal staining may not be specific and may give results in false positive results. This has been seen in several in vitro and in vivo settings.

The main problem of the current senescence research is that a reliable, efficient method for the induction of senescence is missing. An optimal induction system should meet the following requirements. It should be easy to handle and avoid laborious, time-consuming or difficult steps. These requirements are crucial to implement such systems in automation processes. Next, the senescence induction system should be flexible. As mentioned above different stimuli can induce senescence and may provoke different senescence programs. Therefore, the perfect senescence induction system should facilitate an easy replacement of the different senescence stimuli without the need of reconstructing the whole system. Third, the senescence induction system should induce homogenously and reliably the senescence program. This issue is important as a mixture of senescent and non-senescent cells would complicate if not render impossible the elucidation of the senescence signalling pathways. Fourth, as the induced senescence program is dependent on the cell type, the optimal senescence induction system should be transferable to any cell type of choice. Fifth, the senescence induction should not rely on the overexpression of genes but should rather trigger the endogenous senescence program/signalling cascade.

Currently several different systems are available for the induction of senescence. However, all systems have major drawbacks which make them suitable only for very special questions.
(1) Serial passaging as applied first by Hayflick and Moorehead (Hayflick and Moorehead, 1961, supra) leads progressively with increasing passage numbers to a higher percentage of cells that display a senescent phenotype. The disadvantages of this method are that it is very time-consuming and very laborious as the primary cells have to be cultivated and maintained over several months, Secondly, this method relies on the availability of primary cells which is a problem for certain human cell types like rare or difficult to maintain calls. Thirdly, the method of serial passaging is limited as the senescence induction is due to telomere shortening and the induction stimulus cannot be exchanged. Fourthly, serial passaging does not induce homogenously senescence as the primary cells are a mixture of proliferating and senescent cells.
(2) A method described by Chang et al. 2000 (Chang et al., 2000, Proc.NatLAcad.Sci.U.S.A 97: 4291-4296) utilizes the overexpression of p21. In this setting the senescence inducer is transcriptionally regulated (LacSwitch) in an existing tumor cell line (HT1080; fibrosarcoma). Induction of p21 is accomplished by addition of the inducer IPTG which leads to rapid (48h) and homogenous induction of senescence. The disadvantages of this system are that it is fixed to the senescence inducer p21. In addition, the induction of the senescent phenotype requires overexpression of p21. This overexpression might induce artefacts which do not reflect endogenous senescence signalling pathways. Another problem of this method is that it can only be applied to already established (tumor) cell lines which limit its application.
(3) A study by Rubio and colleagues (2002) (Rubio et al., 2002, J.Biol.Chem. 277: 28609-28617) developed a method for the induction of senescence which relies on DNA recombinase mediated excision of an immortalizing gene (hTert). In primary human fibroblasts an immortalizing gene (hTert) was integrated through retroviral infection. This leads to the immortalization of the fibroblasts. hTert was flanked by sequences (loxP-sites) that can be recognized by a DNA recombinase (cre) that facilitates the excision of the sequence between the loxP-sites. The infection of these immortalized fibroblasts with a retrovirus encoding for the DNA recombinase let to the excision of hTert and induced senescence. However, similarly to serial passaging the induction of senescence by this method is laborious, time consuming (>100 days) and inhomogeneous. Another important drawback of this technique is that for the induction of senescence a second genetic modification of the immortalized cells is required. Importantly, the efficiency of this transduction step has to reach nearly 100% as lower transduction efficiencies lead to the overgrowth of the untransduced cells due to their higher proliferation capacity. Since such efficiencies are difficult to obtain complex and time-consuming selection processes have to be performed to yield a purified senescent cell culture.
4) O'Hare et al. 2001 (O'Hare et al., 2001, Proc.Natl.Acad.Sci.U.S.A 98: 646-651) developed a method for the induction of the senescence program that utilizes a temperature sensitive immortalization gene (ts - temperature sensitive SV40 large T antigen) in combination with an unregulated immortalization gene (hTert). The integration of the immortalization genes (tsTAg and hTert) in primary human fibroblasts was accomplished by retroviral transduction. The subsequent cultivation of these cells at the permissive temperature (33°C) led to the activation of both immortalization genes and thereby to the immortalization of the cells. The cultivation of these cells at the non-permissive temperature (39°C) lead to the inactivation of the tsTAg (but not the hTert) and thereby to the induction of senescence within 7 days. This method is accompanied by several drawbacks. The senescence induction is restricted to the tsTAg. As this immortalization is not sufficient to immortalize primary human cells on its own an additional immortalizing gene is needed (hTert). Problematic is the constitutive expression of this additional immortalization gene which in several cases immortalizes primary human cells (see the above mentioned reports). Therefore this approach is restricted to cell types (or cell batches) that are not immortalized by hTert alone. O'Hare used human mammary fibroblasts and endothelial cells which could not be immortalized by hTert alone. Another disadvantage is that for the senescence induction a complex regulation process has to be performed as the cells have to be shifted from one cultivation temperature (33°C) to another (39°C). This is especially a problem for further applications like high throughput screenings which require automation of the cell culture conditions. In addition, for the whole process culture conditions are required that are below or above the physiological temperature. Therefore this method is prone to artefacts which stem from the unphysiological culture conditions.
5) In previous work we demonstrated that transcriptional regulation (Tet-system) of the TAg (SV40 large T antigen) lead to the induction of senescence in primary murine fibroblasts (May et al., 2004, Nucleic Acids Res. 32: 5529-5538). In the activated state (with doxycycline) the cells were immortalized through the activation of TAg. Withdrawal of doxycycline leads to the homogenous induction of senescence within 9 days. The disadvantage of this method is that it is restricted for senescence induction in rodent cells. As mentioned above the senescence programs between murine and human cells differ greatly. In order to exploit the senescence phenomenon for a potential therapy a method for the induction of senescence in human cells would be highly desirable.

The existing methods for inducing senescence have significant drawbacks as mentioned above. Thereby they raise huge technical hurdles which impede a deeper understanding of the senescence program in human cells. But a deeper understanding is essential for targeted therapies that exploit the benefits of senescence while at the same time omit the pitfalls of the senescence program.

In view of the above, there is an ongoing need for methods or preparing and providing senescence cells. Another object of the present invention is to provide transduced immortalized cell lines susceptible to senescence.

A further object of the present invention relates to the provision of a method for screening compounds inducing senescence.

### Summary of the present invention

In a first aspect, a method for the generation of a transduced cell line susceptible to senescence is provided. The senescence is exogenously inducible. Said method comprises the steps of:
a) providing primary cells or cell lines;
b) introducing one or more expression cassettes into the primary cell or cell line of a), said expression cassette(s) comprise(s) at least the following components:
   i) at least one inducible promoter sequence;
   ii) two or more different immortalizing gene sequences which are operably linked with the promoter of i);
   iii) a regulator sequence operably linked with a promoter sequence;
   iv) optionally a selection and/or reporter gene;
c) establishing a transduced primary cell or cell line wherein the expression cassette(s) of b) are introduced into the primary cell or cell line resulting in a transduced cell line tagged with the expression cassette(s) of b);
d) obtaining a transduced cell line tagged with the expression cassettes of b) susceptible to senescence whereby the senescence is exogenously inducible.

In a further aspect, the present invention relates to a transduced immortalized cell line susceptible to senescence obtainable with the method according to the present invention.

In another aspect, the present invention relates to a method for screening molecules influencing senescence of cells comprising the use of the transduced cell lines according to the present invention. Finally, the present invention relates to the use of the transduced cell lines according to the present invention for screening molecules for the production of recombinant proteins as well as kits containing said transduced cell lines in combination with a ligand binding specifically to the regulated gene product influencing the induction of senescence.

### Brief description of the figures

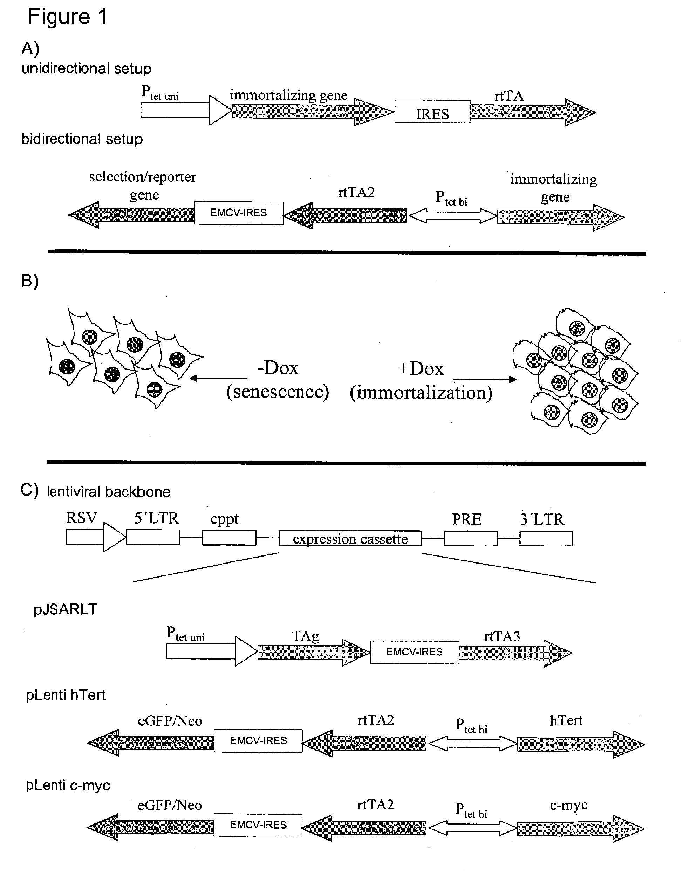

Figure 1 shows a schematic drawing of a first embodiment of the present invention.
Figure 1 a provides a schematic configuration of the expression cassettes introduced into the cell lines.
Figure 1b provides a drawing demonstrating the influence of the ligand binding to the regulator system, here the doxycycline (Dox) molecule. In the presence of Dox the cells are in the immortalized stage while after removal of Dox the cells turns to the senescence stage.
Figure 1c shows different vector setups suitable for the generation of transduced cell lines according to the present invention. Shown is the vector setup which is implemented in a lentiviral vector system.
   pJSARLT refers to the unidirectional tet-dependent promoter, TAg (T-antigen), encephalomyocarditits virus (EMCV)-IRES, reverse transactivator 3.
   pLenti hTert: fusiongene eGFP-Neomycin (reporter molecule), EMCV-IRES, reverse transactivator 2, bidirectional tet-dependent promoter, catalytic subunit of human telomerase (hTert).
   pLenti c-myc: fusiongene eGFP-Neomycin (reporter molecule), EMCV-IRES, reverse transactivator 2, bidirectional tet-dependent promoter, cDNA encoding for human c-myc.

The expression cassettes are introduced into a lentiviral system. These lentiviral systems are self inactivating with a deletion in the 3'LTR which destroys its promoter and enhancer activity. In addition, the HIV-wt promoter from the 5'LTR is replaced by a RSV promoter. To enhance the expression of the transgene cassette a central polypurine tract (cppt) and a hepatitis B posttranscriptional regulatory element (PRE) were also implemented into these vectors. All expression cassettes were cloned between the cppt and the PRE element.

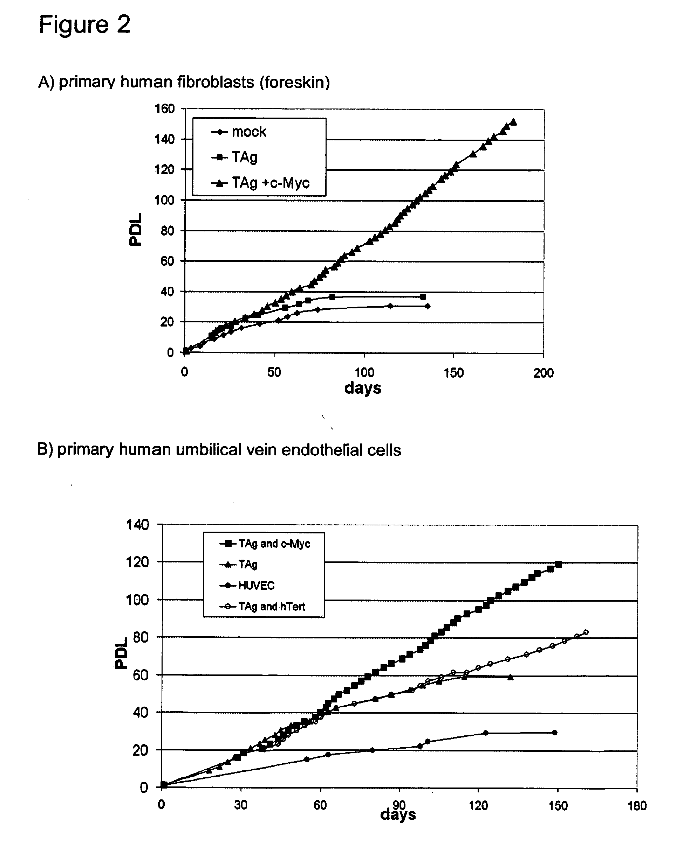

In figure 2 the results for infection of various human primary cells with different lentiviruses are shown. Figure 2a provides the results for primary human fibroblasts derived from the foreskin. The cumulative population doublings in the activated state for the different infections are shown. As controls, the uninfected primary cells were included in the analysis (Mock, circles). The cells that were only infected with the expression cassette regulating TAg are not immortalized but undergo only a few more population doublings compared to the primary cells (TAg, squares) only the culture that was infected with the expression cassettes encoding for TAg and c-myc showed a robust proliferation pattern and are immortalized (TAg + c-myc, triangles). The resulting cell line was named FS4 LTM.

In figure 2b primary human umbilical vein endothelial cells were used as starting material. Said cells, referred to as HUVEC, were infected with the indicated combinations of immortalizing vectors. HUVEC refers to the untransduced primary cells. Again, the cumulative population doublings in the activated state from different cells are shown. The HUVEC cells infected with expression cassettes encoding TAg and c-myc (squares) and TAg and hTert (open circles) were immortalized in contrast to those that were only infected with an expression cassette encoding TAg (triangles). The resulting cell lines were named HUVEC LTM (TAg and c-myc) and HUVEC LTT (TAg and hTert) respectively.

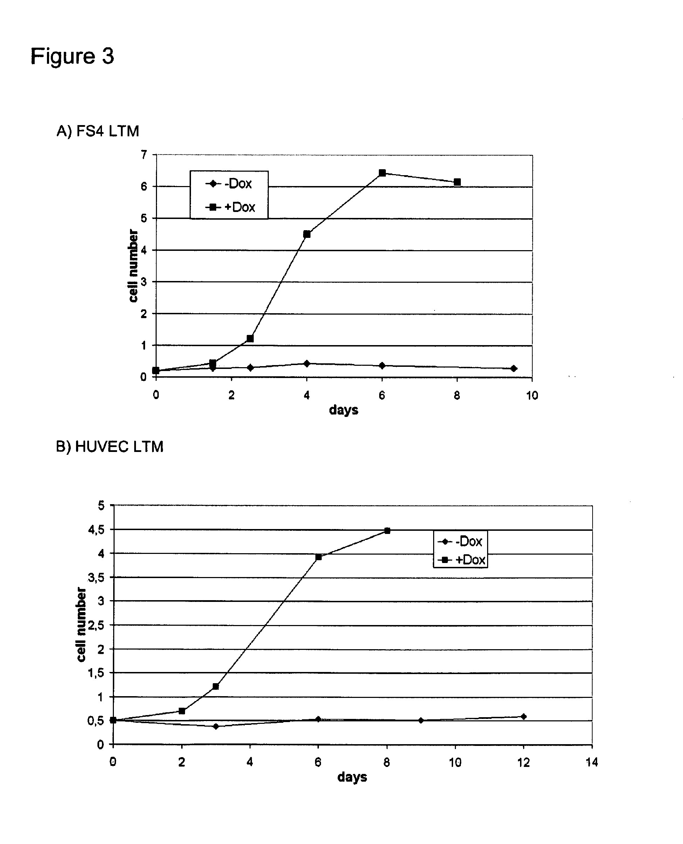

Figure 3 provides a graph showing that the inactivation of the immortalizing genes induced permanent growth arrest. Both cell lines described in figure 2, FS4 LTM and HUVEC LTM, were analysed for proliferation in the presence and absence of Dox. Growth curves of said cells are shown in figures 3a and 3b, cells cultivated with Dox are shown with squares while cells cultivated without Dox as shown with the diamond symbol.

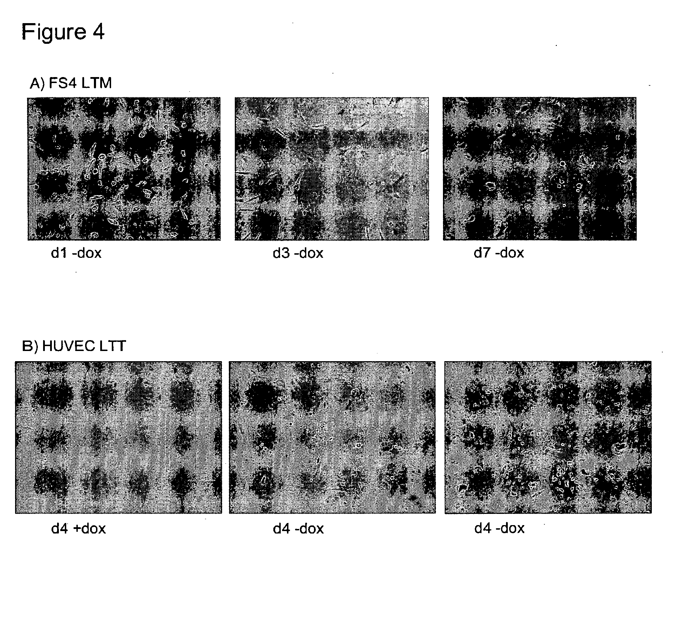

Figure 4 shows the induction of the phenotypic senescence markers after inactivation of the immortalizing genes. Figure 4a shows the results for the FS4 LTM cell line while figure 4b provides the results for the HUVEC LTT cell line. Shown are representative photographs of cultivated cells after 1, 3 or 7 days after Dox withdrawal, respectively, for the FS4 LTM cell line, figure 4a. In figure 4b the HUVEC LTT cells were cultivated in the presence or absence of Dox for 4 days. In figures 4a and 4b the fixed cells were stained for β-galactosidase activity, an accepted marker for senescence.

### Detailed description of the present invention

The present invention relates in a first aspect to a method for the generation of a transduced cell line susceptible to senescence whereby the senescence is exogenously inducible, comprising the steps of:
a) providing primary cells or cell lines;
b) introducing one or more expression cassettes into the primary cell or cell line of a), said expression cassette(s) comprise(s) at least the following components:
   i) at least one inducible promoter sequence;
   ii) two or more different immortalizing gene sequences which are operably linked with the promoter of i);
   iii) a regulator sequence operably linked with a promoter sequence;
   iv) optionally a selection and/or reporter gene;
c) establishing a transduced primary cell or cell line wherein the expression cassette(s) of b) are introduced into the primary cell or cell line resulting in a transduced cell line tagged with the expression cassette(s) of b);
d) obtaining a transduced cell line tagged with the expression cassettes of b) susceptible to senescence whereby the senescence is exogenously inducible.

The term "operably linked" as used herein refers to arrangements of elements wherein the components as described are configured so as to perform their usual function. Thus, a given promoter operably linked to a coding sequence is capable of effecting the expression of the coding sequence when the proper enzyme for transcription of said sequence is present. Furthermore, additional coding sequences can be "operably linked" to the promoter through the incorporation of e.g. internal ribosomal entry sites (IRES) or self processing (cleaving) sequences. Examples for the IRES elements (but not restricted to) are the IRES of the poliovirus or the encephalomyocarditis virus. An example for a cleavage factor is the foot-and-mouth disease virus (FMDV) cleavage factor 2A.

The term "cell line" refers to a population of cells capable of continous growth and division in-vitro under appropriate conditions.

The term "tagged" as used herein is intended to mean a state in which a nucleotide sequence is present in a cell that allows for the transcription and, optionally, the translation of said nucleotide sequence.

The term "regulator system" or "regulator sequence" refers to a sequence or system, respectively, which may act in form of nucleic acid molecules or in its translated form of a polypeptide or protein with a partner molecule (e.g. a promoter responsive to the regulator), thus, altering the activity of the partner molecules, e.g. binding the partner molecules for induction or inhibition of the same.

The present invention provides a new method for the induction of senescence in mammalian preferably human cells. This method combines several enhancements compared to existing methods and therefore will help dramatically to facilitate the elucidation of human senescence. The beneficial features of the method are
(1) a controlled induction of senescence in given culture.
(2) an unlimited supply of cells that can be used for the induction of senescence. Whereby the method creates a homogenous cell culture with constant and reproducible properties.
(3) an easy to handle process for the senescence induction which allow automation of the process.
(4) an induction stimulus which exerts no influence on the cell physiology and thereby does not alter the induced senescence program.
(5) great flexibility as the method is neither fixed to a certain senescence stimulus nor to the number of different senescence stimuli or to a certain cell type.
(6) a senescence induction which relies on endogenous factors and thereby is not driven by overexpression of transgenes which might induce artefacts.

With controlled induction, it is meant herein that the induction of the senescence program is regulated or controlled by exogenous means, thus, the cells start with the senescence program nearly simultaneously.

The method according to the present invention for the induction of senescence in cells, preferably human cells, is based on a complex technique that regulates the immortalization as well as the induction of senescence. In a first step a primary cell, in particular of human origin, is genetically modified with one or more vectors that express all components necessary for the immortalization, regulation and for the induction of senescence. After importing the said vectors into the cell, immortalization is achieved through modulation of the regulation system. Depending on the regulation system immortalization is either achieved through activation or inactivation of the regulation system. This step generates a cell line with constant and reliable properties as all cells are immortalized with the help of the same immortalization stimulus. In addition, this step allows the generation of an unlimited number of cells capable of undergoing senescence. The regulation system can be controlled through different ways as outlined below.

The induction of senescence is accomplished by the inactivation of the immortalization stimulus (stimuli). All cells from the culture respond identical to the inactivation of the immortalization stimulus as they are all established with the same immortalization genes. Therefore the inactivation of the immortalization stimulus leads to (homogenous) precisely controllable induction of the senescence program. Importantly, the senescence is triggered endogenously as the removal of the immortalization stimulus leads to the induction of signalling events that cells encounter when they reach they replication limit.

In a preferred embodiment, the regulation of immortalization/senescence is accomplished through an modulator of transcription. This exogenous transcriptional regulator (transactivator) is transduced inside the cell and can be controlled externally. The transactivator exerts its effects on an exogenous promoter that is also imported inside the cell. This transactivator specific promoter harbours DNA sequences that are recognized by the transactivator and drives the expression of the genes that modulate the immortalization/expansion of the cells. The activity of the transactivator can be controlled externally through the adjustment of the concentration of a ligand capable of binding the transactivator. Therefore, adjusting of the ligand concentration allows the manipulation of the cell state (immortalized/senescent). The ease of this manipulation which is usually accomplished through media modification permits the use of method in automated processes like e.g. high throughput screenings or high content screenings. Due to the external control the method can also be utilized in cell therapy approaches.

Exogenous transcriptional regulation systems are accompanied by a low expression in the uninduced state (basal expression). Therefore it is difficult, time-consuming and laborious to screen for cells that display a strictly regulated expression of the transgene. However we found unexpectedly that despite this basal expression of the transcriptional regulation system a strict control of the immortalization/senescence process is possible even if two or more immortalizing genes are regulated. In the activated state of the transcriptional regulation system immortalization of human cells is induced through the activation of two or more immortalization genes. The inactivation of the transcriptional regulation system led despite the basal expression of the immortalizing genes to the induction of senescence. These findings are independent of the cell type and do not depend on the gene composition that was used for immortalization. This flexibility towards cell type or immortalizing gene combination is a prerequisite to utilize this method for the immortalization/senescence induction in all human cell types and for the induction of all senescence stimuli.

The method for immortalization/senescence induction requires a transcriptional regulation system. In a first aspect, the regulation of this exogenous system relies on the interaction between a transactivator and a corresponding ligand. An important requirement is that the regulation of the transcriptional regulation system does not interfere with the actual research. The regulation of the method according to the present invention using a transactivator is based on the addition of low molecular weight ligands which are usually supplemented as media additives. Due to the low dissociation constant between ligand and transactivator these low molecular weight ligands are supplemented in a concentration range which supports transgene regulation but does not per se exerts any effect on the cell physiology. Therefore all effects observed can be attributed to the immortalization/senescence induction.

In a particular preferred embodiment, the regulation system is a transcriptional regulation system, in particular, a transcription regulation system based on a tet regulation system. The tet regulation system is known in the art, for example, described in Corbel,S.Y. and F.M.Rossi. 2002. Curr.Opin.Biotechnol. 13: 448-452.

Typically, the molecules Tetracycline or Doxocycline are used as the appropriate ligand for the tet system. Other transcription systems include but are not restricted to the AIR-system, the erythromycin system, the PIP system, the Rheoswitch system, the cumate system, the coumermycin system, the NICE system, the T-Rex system.

According to the present invention, two or more different immortalizing gene sequences which are operably linked with a promoter are introduced into the primary cells or cell lines. In a preferred embodiment, the two different immortalizing gene sequences are a combination of any one of the following combinations:
i) early region of the SV40 virus (GeneID: 1489531/ SV40gp6 large T antigen and GeneID: 1489525/ SV40gp7 hypothetical protein) with hTert (GeneID: 7015).
ii) early region of the SV40 virus (GeneID: 1489531/ SV40gp6 large T antigen and GeneID: 1489525/ SV40gp7 hypothetical protein) with c-myc (GeneID: 4609);
iii) early region of the SV40 virus (GeneID: 1489531/ SV40gp6 large T antigen and GeneID: 1489525/ SV40gp7 hypothetical protein) with h-ras (GeneID: 3265) with Bmi1 (GeneID: 648),
iv) Human Papilloma Virus E6 Protein (GeneID: 1489078) with Human Papilloma Virus E7 Protein (GeneID: 1489079),
v) Adenovirus E1A (GeneID: 2652980) with Adenovirus E1B (GeneID: 2652981).
vi) Human Papilloma Virus E7 Protein (GeneID: 1489079) with c-myc (GeneID: 4609),
vii) Adenovirus E1A (GeneID: 2652980) with h-ras (GeneID: 3265).
or functional variants of the above mentioned genes or homologues of the corresponding genes from other mammalian species.

In another preferred embodiments, the immortalizing genes are chosen from: Bmi1 (GeneID: 648), Stat3 (GeneID: 6774), Met GeneID: 4233, Jun GeneID: 3725,c-myc GeneID: 4609, n-myc GeneID: 4613, hox11 GeneID: 3195, oct3/4 GeneID: 5460, β-Catenin GeneID: 1499, bcl2 GeneID: 596, MDM2 GeneID: 4193, myb GeneID: 4602, src GeneID: 6714, Her2 GeneID: 2064, genes of the E2F family: E2F1 GeneID: 1869; E2F2 GeneID: 1870; E2F3 GeneID: 1871; E2F4 GeneID: 1874; E2F5 GeneID: 1875; E2F6 GeneID: 1876; E2F7 GeneID: 144455; E2F8 GeneID: 79733 , hes1 GeneID: 3280, hey1 GeneID: 23462, cyclinD1 GeneID: 595, hras GeneID: 3265, Sox2 GeneID: 6657, early region of the SV40 virus (GeneID: 1489531/ SV40gp6 large T antigen and/or GeneID: 1489525/ SV40gp7 hypothetical protein, Hoxa9 GeneID: 3205, Hoxb8 GeneID: 3218, hTert GeneID: 7015, Human Papilloma Virus E6 Protein (GeneID: 1489078), Human Papilloma Virus E7 Protein (GeneID: 1489079), pim1 GeneID: 5292, pim2 GeneID: 11040, Nanog GeneID: 79923, TBX2 GeneID: 6909; Raf1 GeneID: 5894, MAP3K1) GeneID: 4214, mos GeneID: 4342, ets1 GeneID: 2113, ER81 GeneID: 2115, ets2 GeneID: 2114, vGPCR (homologue of the human interleukin-8 (IL-8) receptor) (GeneID: 935079); Rex1 (ZFP42) GeneID: 132625, ID2 GeneID: 3398, ID1 GeneID: 3397, Fos GeneID: 2353, k-ras GeneID: 3845, HoxB4 GeneID: 3214, Adenovirus E1A (GenoID: 2652980), Braf GeneID: 673, Adenovirus E1B (GeneID: 2652981), KLF4 GeneID: 9314, Bcl-XI (BCL2L1) GeneID: 598, LMO2 GeneID: 4005, Meis1 GeneID: 4211, Hesx1 GeneID: 8820, Zic3 GeneID: 7547, LIN28 GeneID: 79727 and functional variants of the above mentioned genes or homologues of the corresponding gene from other mammalian species.

In a second embodiment for the immortalization process, typical tumor suppressive genes are downregulated to effect immortalization. That is, typical tumor suppressive gene(s) like the tumor suppressive genes p53, p16, p14, pRB (p105), p107, p130, Pten and the like are downregulated with known techniques. Said techniques encompass the use of corresponding ligands based on siRNA, miRNA, dominant negative mutants, ribozymes and antisense molecules directed against one or more of known tumor suppressive genes or other molecules downregulating or suppressing the activity of tumor suppressive gene(s) or other molecules downregulating the proliferation of cells.

The method of using siRNA and miRNA is well known in the art, e.g. as described by Pei,Y. and T.Tuschl. 2006. Nat.Methods. 3: 670-676 and Chang et al., 2006, Nat. Methods. 3: 707-714.

In order to achieve immortalization either immortalizing genes or the downregulation of tumor suppressor genes can be applied. But the immortalization process can also be induced through a combination of immortalizing gene(s) and the downregulation of tumor suppressor gene(s).

For the human system it has been recognized by the present inventors that at least two immortalizing gene sequences are necessary to allow the generation of a transduced cell line, which has been immortalized and which is susceptible to senescence when the appropriate stimuli is given. For example, the induction of senescence is accomplished by the inactivation of the immortalizing stimulus.

Thus, in a preferred embodiment, the present invention relates to a method for the generation of human transduced cell lines susceptible to senescence derived from human primary cell or cell lines.

In a further aspect, the present invention relates to the transduced cell line obtainable with the method according to the present invention. Said transduced immortalized cell line are characterized in containing at least two immortalizing genes which are operably linked with a promoter whereby said promoter is an inducible promoter. Furthermore, the transduced immortalized cell line according to the present invention contains a regulator sequence operably linked with a promoter sequence and, optionally, a selection and/or reporter gene.

Said transduced cell lines are characterized in displaying typical phenomena associated with senescence when inactivating the immortalizing stimuli. That is, said transduced cell line according to the present invention may display a senescence associated beta galactosidase staining, a slower cell proliferation in the senescent state compared to the immortalized state or a permanent growth arrest in the senescent state, the presence of senescence associated heterochromatin foci, senescence associated change in cell morphology, senescence associated increase in p161nk4a expression, increase in p21 expression, increase in p53 expression, increase in p15INK4b expression, increase in Dec1 expression, increase in DcR2 expression, senescence associated focal nuclear staining of HP1, senescence associated weak expression of Ki-67, senescence associated focal nuclear staining of K9M-H3(methylation of Lys9 on histone H3), phosphorylated histone H2ax (γ-H2AX), senescence associated weak DNA replication.

These transduced cell lines obtainable according to the method of the present invention may be utilized in methods for screening molecules influencing the senescence of cells. That is, in another embodiment, the present invention relates to a method for screening molecules influencing senescence of cells comprising the step of
a) providing a transduced cell line according to the present invention;
b) providing a molecule to be tested;
c) incubating a sample containing the cell line of a) in the presence and in the absence of the molecule of step b);
d) determining the status of senescence in each of the two samples;
e) identifying molecules altering senescence in said transduced cell line.

Said method is particularly useful to be performed in high throughput screening method allowing the automation of the screening process. The molecules identified with the method for screening molecules influencing the senescence of cells according to the present invention may be useful for induction or inhibition of senescence cells. That is, said compounds identified with the method according to the present invention may be formulated into pharmaceuticals for the treatment of various disease, disorders or conditions associated with the senescence of cells, like arthritis, atherosclerosis, cancer, metastasis, psoriasis, diabetes, infections (e.g. bacterial, viral), degenerative disorders (e.g. Alzheimer's disease), ulcers.

In a preferred embodiment, the status of senescence determined in each of the two samples is one or more of the following markers:
senescence associated β-galactosidase activity, senescence associated heterochromatin foci, senescence associated change in cell morphology, senescence associated increase in p16Ink4a expression, increase in p21 expression, increase in p53 expression, increase in p15INK4b expression, increase in Dec1 expression, increase in DcR2 expression, senescence associated focal nuclear staining of HP1, senescence associated weak expression of Ki67, senescence associated focal nuclear staining of K9M-H3(methylation of Lys9 on histone H3), phosphorylated histone H2ax (γ-H2AX), senescence associated weak DNA replication.

In addition, the present invention provides in a further embodiment kits comprising the transduced cell line according to the present invention in combination with a formulation that contains the appropriate ligand binding specifically to the regulator gene or gene product.

As indicated above, said ligand may be a molecule binding to the regulator gene product, like the transactivator, e.g. tet. Said ligand may be for example doxycycline or other molecules known as ligands for the tet regulation system.

Finally, the present invention relates to the use of the transduced cell line according to the present invention for screening molecules. In this setting a molecule like e.g, a chemical entity, a peptide, a protein or a nucleotide sequence is incubated with the transduced cell line and the influence of this molecule is determined. In addition, the transduced cell line according to the present invention may be used for the production of recombinant proteins. In this setting the present invention allows to establish the required cell number for the protein production process with the initial immortalization phase. For the production of the recombinant protein senescence is induced and allows a prolonged production phase. The transduced cell line described in this invention may also be used in cell therapy approaches. In this setting the desired cell type is expanded in the first step. The second step comprises the activation of the senescence stimulus and the use of the cells for cell transplantations. Alternatively the cells may also be encapsulated and used for the therapy (e.g. bloartificial organs). For the cell therapy either an in vivo or an ex vivo use of the senescent cells can be applied. An additional use of the transduced cell line described in this invention is to determine the influence of a molecule on the cell physiology. That is in a first step the desired cell type is expanded. In a second step senescence is induced and a molecule is incubated with the senescent cells. This step allows to measure parameters like adsorption (e.g. to determine the uptake of a molecule), distribution (e.g. to determine how a molecule is distributed), metabolism (e.g. to determine if and how a molecule is metabolized), excretion (e.g. to determine the excretion mechanisms) and toxicity (e.g. to determine the influence on the cell vitality). Another use of the transduced cell line described in this invention is its use in the setup of in vitro organ cultures. In a first step the desired cell type(s) is/are expanded. In a second step senescence is induced and the cells are in vitro reassembled into so called in vitro organ cultures. For this step one or more cell types can be applied.

For the generation of the transduced cell line according to the present invention, the expression cassette may optionally contain a selection and/or reporter gene encoding a selection marker or reporter molecule, accordingly.

In this connection, the term "selection marker" as used herein is intended to mean a marker which enables discrimination of moieties, like cells, which may or may not contain said selection marker. For example, a selection marker may be the expression of a specific molecule, i.e. a reporter molecule, a resistance marker, like a marker conferring drug resistance to a cell, etc. The selection marker allows for simpler preparation, manufacturing, characterization or testing of the cell or cell line. The reporter gene or reporter molecule refers to a typical reporter or marker known in the art. For example, said reporter gene may be a gene encoding GFP or other marker molecules known in the art, like cellular and extracellular expression markers, fluorescence markers, beta galactosidase, secreted alkaline phosphatase and cell surface markers. That is, the reporter groups are in general groups that make the introduced cell line distinguishable from the non-transduced cell line. This distinguishment can be either effected by selecting the reporter gene from the group of directly or indirectly detectable groups. Directly detectable groups are for example fluorescence compounds, like fluorescein, or its derivatives such as hexachlorofluorescine and hexafluorofluorescine, other fluorescent compounds or fluorescent particles. Of course, other markers may be used, like radioactive isotopes of carbon, hydrogen, nitrogen, etc.

Establishing the transduced primary cell or cell line wherein the expression cassette(s) are introduced, thus, resulting in a transduced cell line, tagged with the expression cassette(s) is effected based on selection processes, e.g. based on the respective selection or reporter molecules. Selection may be effected based on negative or positive selection for said selection or reporter molecules. Selection may also be performed through the growth advantage of the transduced cells compared to the unmodified ones.

In a preferred embodiment, the transduction is effected through infection or transfection. In a preferred embodiment, transduction is effected with virus based systems, e.g. the lentiviral based system. Useful expression cassettes utilized in the lentiviral system are described for example in WO 2006/048228 which is herein incorporated by reference. For example, the expression cassette contains additionally a 5'LTR and a 3'LTR. Further; the expression cassette may additionally contain a central polypurine tract (cppt) and/or a post-transcriptional regulatory element (PRE) (e.g. from the hepatitis B virus or the woodchuck hepatitis virus). Further, preferably the expression cassette harbours a modified 3'LTR or both the 3'LTR and 5'LTR are modified. For example the expression cassette comprises a RSV promoter which is implemented in the 5'LTR. That is, in a preferred embodiment, the expression cassette(s) is/are introduced through lentiviral infection. Alternatively, the expression cassettes may be introduced through plasmid transduction which can be e.g. achieved through electroporation, calcium phosphate transfection or lipofection. The expression cassette may be integrated into the chromosome or may be present as a minicircle or in other episomal forms. Further, the expression cassette may be implemented into a bacterial artificial chromosome.

The invention will be described further by reference to the examples. It is clear that said examples are not intended to limit the scope of the present invention.

### Examples

### Example 1 Production of the lentiviral particles

For the transduction of the human primary cells lentiviral infection was used. For this purpose different lentiviral expression plasmids were constructed that are so-called third generation self-inactivating lentiviral vectors. In these lentiviral plasmids the viral sequences have been successively reduced. In this vector generation not only the accessory genes (vpr, nev), rev and gag/pol are deleted but also the viral LTRs are modified so that the viral promoter/enhancer sequence is deleted. Furthermore in the 5'LTR a RSV promoter is included instead of the wtHIV promoter. In order to increase the titer additional elements (1) the central polypurine tract (cppt) and (2) the posttranslational regulatory element from the Hepatitis B virus (PRE) were included in the lentiviral vector. These elements were arranged 5' (cppt) and 3' (PRE) to the expression cassette.

A schematic drawing of the principle of the present method is shown in figure 1.
This overview exemplifies the different vector setups suitable of generating cells capable of inducing senescence in human cells. In the following examples the tet-on system is used as the regulation system to control the immortalization/senescence process in human cells. As an example for the immortalization the combination of the immortalizing genes hTert and TAg or c-myc and TAg were used. However, for the present method any transcriptional regulation system can be employed and several different expansion/immortalization genes can be used.

As shown generally in figure 1a, in the first setup a bicistronic mRNA is expressed from a unidirectional tet-dependent promoter. Upon induction the expression of an immortalizing gene as well as the expression of the reverse transactivator is activated. The expression of the transactivator is facilitated through an internal ribosomal entry site (IRES) which links both genes (unidirectional setup). The second setup (bidirectional setup) employs a bidirectional tet-dependent promoter which drives the expression of two opposing mRNAs. One mRNA codes for an immortalizing gene, while the second is encoding for the transactivator and a selection/reporter construct.

For the induction of immortalization of primary human cells the combined induction of two immortalizing genes has to occur. The addition of Dox leads to the expression of the introduced expression cassettes and thereby to the immortalization of primary human cells. Withdrawal of Dox leads in turn to the inactivation of the immortalization genes and thereby to the induction of senescence.

All used expression cassettes were implemented into a third generation lentivirus. These lentiviruses are self inactivating with a deletion in the 3'LTR which destroys its promoter/enhancer activity. In addition the HIV-wt promoter from 5'LTR is replaced by a RSV promoter. To enhance the expression of the transgene cassette a central polypurine tract (cppt) and a Hepatitis B post-transcriptional regulatory element (PRE) was also implemented into these vectors. All expression cassettes were cloned between the cppt and the PRE-element.

The expression cassettes were comprised of the following components (listed from 5'to 3') (see figure 1 c)
pJSARLT: unidirectional tet-dependent promoter, first cistron: TAg, EMCV-IRES, second cistron: reverse transactivator 3 (Das et al., 2004, J.Biol.Chem. 279: 18776-18782)
pLenti hTert: (in antisense to the lentiviral vector) second cistron: fusiongene eGFP-Neomycin, EMCV-IRES, first cistron: reverse transactivator 2, bidirectional tet-dependent promoter, (in sense to the lentiviral vector) first cistron: hTert
pLenti c-myc: (in antisense to the lentiviral vector) second cistron: fusiongene eGFP-Neomycin, EMCV-IRES, first cistron: reverse transactivator 2 (Urlinger et al., 2000, ProC.NatLAcad.Sci.U.S.A 97: 7963-7968), bidirectional tet-dependent promoter, (in sense to the lentiviral vector) first cistron: c-myc.

For the production of the viral particles a transient transfection protocol was used that is based on calcium phosphate precipitation of four different plasmids. The production of the lentiviral particles was done in HEK293T cells with lentiviral helper plasmids from the ViraPowerTM Lentiviral Expression System (Invitrogen). A day before transfection HEK293T cells were plated at a cell density of ∼20000 cells per cm². For the calcium phosphate precipitation the four plasmids encoding the helper functions gag/pol (pLP1), rev (pLP2) and the VSVg (pVSVg) surface protein along with one of the transfer plasmids (pJSARLT, pLenti c-myc, pLenti hTert) were mixed. For a transfection of a culture dish with an area of 140cm² the following amounts of the different plasmids were used:
14 µg pLP1 (gag/pol), 4,6 µg pLP2 (rev); 7,8 µg pLPIVSVg (VSVg), 20 µg transfer plasmid (pJSARLT, pLenti c-myc, pLenti hTert).

The different plasmids were resuspended in 500µl of 250 mM CaCl₂ solution and then this mixture was dropwise added to 500 µl of HEBS solution under continuous vortexing and then kept for 10 minutes at room temperature for precipitation. This suspension was added to the culture medium of the HEK293T cells. The following day the media was aspirated and 15 ml fresh media was added. 24 h later the supernatant containing the lentiviral particles was collected. For a second production again 15 ml fresh media was added to the producer cells and after 24h the supernatant containing the lentiviral particles were collected. The lentivirus containing supernatant was filtrated with a 0,45 µm filter to get rid of cell debris and stored at - 70°C until use.

### Example Establishment of human cell line capable of inducing senescence

The cells were maintained during the whole process at 37 °C in a humidified atmosphere with 5% CO₂. The human primary foreskin fibroblasts (FS4) were maintained in DMEM (Gibco-BRL) supplemented with 10% foetal calf serum, 2 mM L-glutamine, penicillin (10 U/ml) and streptomycin (100 µg/ml). Human umbilical vein endothelial cells (HUVECs) were purchased from Promocell and cultivated in endothelial-cell basal medium EBM (Lonza) supplemented with EGM-MV single quots (Lonza). Endothelial cells were cultivated in gelatine-coated tissue-culture flasks. For the activation of the immortalization Dox (doxycycline hyclate, Sigma) was added to the culture media at a concentration of 2 µg/ml.

The immortalization procedure was divided into two different steps. In the first round of infection the lentiviruses transducing the expression cassette JSARLT were used to infect the primary human fibroblasts or the primary human umbilical vein endothelial cells, For this purpose 1*10⁵ cells were plated per 9 cm² dish a day before infection (∼10000 cells per cm²). For the infection the culture media of the cells was aspirated. Afterwards 1ml of undiluted viral supernatant was supplemented with 8 µg/ml polybrene and added to the cells for 8h. Afterwards the viral supernatant was discarded and a second round of infection with the same conditions was performed for 12h. The next day the viral supernatant was discarded and the cells were cultivated with media supplemented with 2µg/ml Dox. The cells were cultivated for three to five days before the second infection round was performed. For this purpose 1*10⁶ cells were plated per 9 cm² dish a day before infection. For the infection the culture media was aspirated and 1ml of undiluted viral supernatant supplemented with 8µg/ml polybrene was added to the cells for 12h. In this round of infection either lentiviruses transducing Lenti hTert or Lenti c-myc were used. This infection cycle was repeated three times. Two days after the last infection, selection pressure was applied (0,2-0,4µg/ml G418). During the whole process of establishing a cell line the media was supplemented with 2µg/ml dox and renewed every 4 days. The resulting G418 resistant colonies were pooled and further expanded. For the establishment of the cell lines the cells were passaged when they reached confluency. In the beginning of the immortalization process the cells were splitted with a ratio of 1 to 5. After the cells adapted a robust proliferation behaviour (meaning that they reached confluency within three days after plating) the splitting ration was adjusted to 1 to 10.

The results of the immortalization of human primary cells are shown in figure 2.

In figure 2a the cumulative population doublings in the activated state for the different infections are shown. As controls the untransduced primary cells were included in the analysis (mock, circles). The cells that were only infected with the expression plasmid regulating TAg are not immortalized but undergo only a few more population doublings compared to the primary cells (TAg, squares). Only the culture that was infected with the expression plasmids encoding for TAg and c-myc showed a robust proliferation pattern and are immortalized (TAg + c-myc, triangles). This cell line was named FS4 LTM.

In figure 2b, primary human umbilical vein endothelial cells (HUVEC) were infected with the indicated combinations of immortalizing vectors (TAg+ c-myc; TAg+ hTert, TAg, untransduced). In the graph the cumulative population doublings in the activated state (+Dox; 2 µg/ml) from different cells are shown. The primary cells (HUVEC, black circles) and the culture that was transduced with TAg (black triangles) alone were not immortalized. The cultures that were infected with the TAg + c-myc (black squares) and TAg + hTert (open circles) combinations showed a robust proliferation and were immortalized. The resulting cell lines were named HUVEC LTM (TAg + c-myc) and HUVEC LTT (TAg + hTert).

### Example Inducing a permanent growth arrest of the established cell lines

For the determination of the growth arrest an exponentially proliferating cell line was splitted and the cell number was determined using a CASY Counter (Schärfe Systems, GmbH). For the analysis of the proliferation pattern of immortalized and senescent cells 2*10⁴ cells were plated per 9 cm² dish (-2000 cells per cm²) and cultivated with (2µg/ml) and without Dox. The media was renewed every four days throughout the experiment. At the indicated time points the cells were washed with PBS, trypsinized and counted with a CASY Counter. Per time point three independent samples were counted. To exclude cell debris and cell aggregates from the analysis only particles with a size between 10 µm and 50 µm were counted.

In figure 3, the inactivation of the immortalizing genes induces a permanent growth arrest. For this analysis a growth curve was established for which the cell number was determined at indicated time points. For this analysis the samples were plated in triplicate and the mean values were determined.
A) FS4 LTM, Cells cultivated with dox (squares), cells cultivated without dox (diamond)
B) HUVEC LTM. Cells cultivated with dox (squares), cells cultivated without dox (diamond)

### Example Induction of SA-β-Gal activity In celts cultivated without dox

For the analysis of the SA-β-Gal activity exponentially proliferating cells were split, counted and plated without dox at different cell densities in the range between 1500 and 15000 cells per cm². After the indicated time points the cells were washed with PBS and afterwards fixed for 5 min at room temperature with formaldehyde and glutardialdehyde (9,6 % formaldehyde, 0,4% glutardialdehyde dissolved in PBS). After the fixation the cells were washed twice with PBS before they were stained over night with 5 mM K₄[Fe(CN)6] (potassium hexacyanoferrate (II)), 5 mM K₃[Fe(CN)₆] (potassium hexacyanoferrate(III)), 2 mM MgCl₂, 150 mM NaCl, 40 mM NaH₂PO₄, 1 mg/ml X-gal (5-bromo-4-chloro-3-indolyl-beta-D-galactopyranoside) (dissolved in dimethylformamid) and 40 mM citric acid pH 6,0. Results are shown in figure 4.

Figure 4 shows that phenotypic senescence markers are induced after inactivation of the immortalizing genes.

That is, to determine if the growth arrest observed in the cell lines FS4 LTM, HUVEC LTM and HUVEC LTT is senescence additional markers (in addition to the growth arrest) were analyzed. For this analysis cells were plated in the uninduced state (without Dox). After the indicated times the cells were washed with PBS and fixed with formaldehyde, glutardialdehyde. Following the fixation the cells were stained over night for an endogenous β-galactosidase activity (SA-β-gal - senescence associated β galactosidase activity) which is an accepted marker for senescence. Shown are representative pictures of FS4 LTM (A) and HUVEC LTT (B) cells, as a control HUVEC LTT cells were also stained in the immortalized state. Of note, the cells that were cultivated without dox changed their morphology. This process starts a day after the dox withdrawal. The difference in morphology between both the immortalized and senescent state can be clearly observed after 4 days. This change of the morphology to a more flattened and spread phenotype is a typical feature of senescent cells.
A) For the FS4 LTM cells a time course experiment was performed. The SA-β-gal-activity was analyzed 1, 3 and 7 days after dox withdrawal. After one day of dox withdrawal there was no sign of any SA-β-gal activity. This result is in line with reports that showed that the appearance of the SA-β-gal activity is not seen through the initiation of senescence. After three days the SA-β-gal activity could be detected and then the intensity continued to increase to day 7, figure 4a.
B) The HUVEC LTT cells were cultivated with (2µg/ml) and without dox for four days. As expected no SA-β-gal activity could be detected in the immortalized cells. In contrast the uninduced cells showed a strong homogenous staining pattern, figure 4b.

## Claims

1. A method for the generation of a transduced cell line susceptible to senescence whereby the senescence is exogenously inducible, comprising the steps of:
a) providing primary cells or cell lines;
b) introducing one or more expression cassettes into the primary cell or cell line of a), said expression cassette(s) comprise(s) at least the following components:
i) at least one inducible promoter sequence;
ii) two or more different immortalizing gene sequences which are operably linked with the promoter of i);
iii) a regulator sequence operably linked with a promoter sequence;
iv) optionally a selection and/or reporter gene;
c) establishing a transduced primary cell or cell line wherein the expression cassette(s) of b) are introduced into the primary cell or cell line resulting in a transduced cell line tagged with the expression cassette(s) of b);
d) obtaining a transduced cell line tagged with the expression cassettes of b) susceptible to senescence whereby the senescence is exogenously inducible.

2. The method according to claim 1 wherein the regulator sequence in step iii) is a transactivator sequence.

3. The method according to claim 1 or 2 wherein a combination of immortalization genes are chosen from i) early region of the SV40 virus with hTert, ii) early region of the SV40 virus with c-myc; iii) early region of the SV40 virus with h-ras with Bmi1, iv) Human Papilloma Virus E6 Protein with Human Papilloma Virus E7 Protein, v) Adenovirus E1A with Adenovirus E1B, vi) Human Papilloma Virus E7 Protein with c-myc, vii) Adenovirus E1A with ras and functional variants of the above mentioned genes or homologues of the corresponding genes from other mammalian species.

4. The method according to claim 1 or 2, wherein the immortalizing genes are chosen from Bmi1, Stat3, Met, Jun, c-myc, n-myc, hox11, oct3/4, β-Catenin, bcl2, MDM2, myb, src, Her2, genes of the E2F family, hes1, hey1, cydinD1, hras, Sox2, early region of the SV40 virus, Hoxa9, Hoxb8, hTert, Human Papilloma Virus E6 Protein, Human Papilloma Virus E7 Protein, pim1, pim2, Nanog, TBX2 Raf, Mek (MAP kinase/ERK kinase), mos, ets1, ER81, ets2, vGPCR (homologue of the human interleukin-8 (IL-8) receptor); Rex1, ID2, ID1, Fos, k-ras, HoxB4, Adenovirus E1A, Braf Adenovirus E1B, KLF4, Bcl-XI, LMO2, Meis1, Hesx1, Zic3, LIN28 and functional variants of the abovementioned genes or homologues of the corresponding gene from other mammalian species.

5. The method according to claim 1 or 2, wherein for immortalization at least one of the following tumor suppressor gene(s) are downregulated p53, p16, p14, pRB (p105), p107, p130, Pten.

6. The method according to any one of the preceding claims wherein the induction is accomplished through a transcriptional regulation system.

7. The method according to any one of the preceding claims wherein the induction is accomplished through a tet regulation system.

8. The method according to any one of the preceding claims wherein the introduction of the expression cassette is performed by transfection, electroporation or an infection.

9. The method according to any one of the preceding claims wherein the primary cell or cell line is of human origin.

10. A transduced immortalized cell line obtainable with a method according to any one of claims 1 to 9

11. The transduced cell line according to claim 10 **characterized in** having a senescence associated β-galactosidase activity in the senescence state.

12. The transduced cell line according to claim 11 **characterized** through at least one of the following features (i) a change of cell morphology upon senescence induction, (ii) the induction of heterochromatin foci in the senescence state, (iii) a block of proliferation in the senescent state or (iv) a slower proliferation in the senescence state compared to the immortalized state.

13. A kit comprising a transduced cell line according to any one of claims 10 to 12 and a ligand binding specifically to the regulator gene or gene product.

14. A method for screening molecules influencing senescence of cells comprising the step of
a) providing a transduced cell line according to any one of claims 10 to 12;
b) providing a molecule to be tested;
c) incubating a sample containing the cell line of a) in the presence and in the absence of the molecule of step b);
d) determining the status of senescence in each of the two samples;
e) identifying molecules altering senescence in said transduced cell line.

15. The method according to claim 14 wherein the status of senescence is determined by one or more of the following markers: senescence associated β-galactosidase activity, senescence associated heterochromatin foci, senescence associated change in cell morphology, senescence associated increase in p16Ink4a expression, increase in p21 expression, increase in p53 expression, increase in p15INK4b expression, increase in Dec1 expression, increase in DcR2 expression, senescence associated focal nuclear staining of HP1, senescence associated weak expression of KI67, senescence associated focal nuclear staining of K9M-H3(methylation of Lys9 on histone H3), phosphorylated histone H2ax (γ-H2AX),senescenre associated weak DNA replication.

16. The use of a transduced cell according to any one of claims 10 to 12 for screening molecules, production of recombinant proteins, in cell therapy, for determining the influence of a molecule on the cell physiology, or in in vitro organ cultures.
